# EUROPÄISCHE PATENTSCHRIFT

(11) **EP 0 600 372 B1**
(45) Veröffentlichungstag und Bekanntmachung des Hinweises auf die Patenterteilung: **05.02.1997**
(21) Anmeldenummer: 93118993.0
(22) Anmeldetag: 25.11.1993
(51) Int. Cl.: C07K 14/62, C07K 1/113

(54) **Verfahren zur Gewinnung von Proinsulin mit korrekt verbundenen Cystinbrücken**
Process for the preparation of proinsulin with correctly linked cystin bridges
Procédé de préparation de proinsuline à ponts de cystine liés correctment

(30) Priorität: 02.12.1992 DE 4240420
(43) Veröffentlichungstag der Anmeldung: 08.06.1994
(73) Patentinhaber: HOECHST AKTIENGESELLSCHAFT, 65929 Frankfurt am Main (DE)
(72) Erfinder: Obermeier, Rainer, Dr., D-65795 Hattersheim am Main (DE); Gerl, Martin, Dr., D-65830 Kriftel/Ts. (DE); Ludwig, Jürgen, D-63636 Brachttal (DE); Sabel, Walter, D-65520 Bad Camberg (DE)

(56) Entgegenhaltungen:
- EP-A- 0 347 781
- WO-A-91/03550
- CHEMICAL ABSTRACTS, Band 114, Nr. 11, 18. MUrz 1991, Columbus, Ohio, USA ALEKSYUK, I.V. et al. "Use of thiol sorbents for pro- duction of recombinant human proinsulin" Seite 567, Nr. 99 871s; & Bioorg. Khim. 1990, 16(12), 1683-6

## Beschreibung

Humaninsulin ist ein Protein mit zwei Aminosäureketten mit zusammen 51 Aminosäureresten. In den beiden Aminosäureketten kommen 6 Cysteinreste vor, wobei je zwei Cysteinreste über eine Disulfidbrücke untereinander verbunden sind. In biologisch aktivem Humaninsulin sind die A- und B-Ketten über zwei Cystinbrücken miteinander verbunden, und eine weitere Cystinbrücke kommt in der A-Kette vor. Innerhalb eines Humaninsulinmoleküls gibt es statistisch gesehen 15 Möglichkeiten zur Ausbildung von Disulfidbrücken. Im biologisch wirksamen Humaninsulin kommt nur eine der 15 Möglichkeiten vor. Die folgenden Cysteinreste sind im Humaninsulin miteinander verknüpft:
A 6 - A 11
A 7 - B 7
A 20 - B 19

Die Buchstaben A oder B stehen für die jeweiligen Insulinketten, die Zahl für die Position des Aminosäurerestes, die vom Amino- zum Carboxylende der jeweiligen Aminosäurekette gezählt wird. Disulfidbrücken können auch zwischen zwei Humaninsulinmolekülen ausgebildet werden, so daß leicht unübersehbar viele verschiedene Disulfidbrücken entstehen können.

Ein bekanntes Verfahren zur Herstellung von Humaninsulin beruht auf der Verwendung von humanen Proinsulin. Humanes Proinsulin ist ein Protein mit einer linearen Aminosäurekette aus 86 Aminosäureresten, wobei die A- und B-Ketten des Humaninsulins über ein C-Peptid mit 35 Aminosäureresten miteinander verbunden sind. Die Ausbildung der in Humaninsulin vorkommenden Disulfid-brücken erfolgt über ein Zwischenprodukt, wobei die Cysteinreste des Humaninsulins mit einer Schwefelschutzgruppe, z.B. einer S-Sulfonat (-S-SO₃⁻)-Gruppe versehen sind (EP 0 037 255). Ferner ist ein Verfahren zur Gewinnung von Proinsulin mit korrekt verbundenen Cystinbrücken bekannt (Biochemistry, 60, (1968), Seiten 622 bis 629), das von Proinsulin aus Schweinen ausgeht, bei dem die Cysteinreste als Thioreste (-SH) vorliegen. Unter dem Begriff "korrekt verbundenen Cystinbrücken" versteht man die Disulfidbrücken, die in biologisch aktiven Insulin aus Säugetieren vorkommen.

Gentechnische Verfahren erlauben es, menschliches Proinsulin oder Proinsulin, das eine von Humaninsulin abweichende Aminosäuresequenz und/oder Aminosäurekettenlänge hat, in Mikroorganismen herzustellen. Die von gentechnisch veränderten Escherichia-coli-Zellen hergestellten Proinsuline haben keine korrekt verbundenen Cystinbrücken. Ein Verfahren zur Gewinnung von Humaninsulin mit E. coli (EP 0 055 945) beruht auf folgenden Verfahrensschritten:
Fermentation der Mikroorganismen - Zellaufschluß - Isolierung des Fusionproteins - Bromcyanspaltung des Fusionproteins - Isolierung des Spaltprodukts mit der Proinsulinsequenz - Schutz der Cysteinreste von Proinsulin durch S - Sulfonat-Gruppen - Ausbildung der korrekt verbundenen Cystinbrücken - Sulfitolyse - Entsalzen des Proinsulins - Chromatographische Reinigung des Proinsulins mit korrekt verbundenen Cystinbrücken - Konzentrierung der Proinsulinlösung - Chromatographische Reinigung der konzentrierten Proinsulinlösung - Enzymatische Spaltung des Proinsulins zur Gewinnung von Humaninsulin - Chromatographische Reinigung des entstandenen Humaninsulins.

Nachteile dieses Verfahrens sind die Anzahl der Verfahrensschritte und die Verluste bei den Reinigungsschritten, die zu einer geringen Ausbeute an Insulin führen. Wegen des vielstufigen Verfahrensweges müssen erhebliche Verluste bei dieser Verfahrensweise in Kauf genommen werden. Von der Stufe des isolierten Fusionproteins über Bromcyanspaltung, Sulfitolyse und Reinigung des Proinsulins muß mit einem bis zu 40%igem Verlust von Proinsulin gerechnet werden (EP 0 055 945). Ähnlich hohe Verluste können im Verlauf der nachfolgenden Reinigungsschritte bis zum Endprodukt auftreten.

Ausbeutesteigerungen bei der gentechnischen Herstellung von Humaninsulin oder Insulinderivaten lassen sich dann erzielen, wenn die Zahl der notwendigen Verfahrensschritte wesentlich verringert werden kann.

Aufgabe der vorliegenden Erfindung war es, ein Verfahren zur Gewinnung von Proinsulin mit korrekt verbundenen Cystinbrücken der Proinsulinaminosäurekette zu entwickeln, bei dem weniger Verfahrensschritte nötig sind und insgesamt geringere Reinigungsverluste auftreten.

Es wurde nun ein Verfahren zur Gewinnung von Proinsulin der Formel I 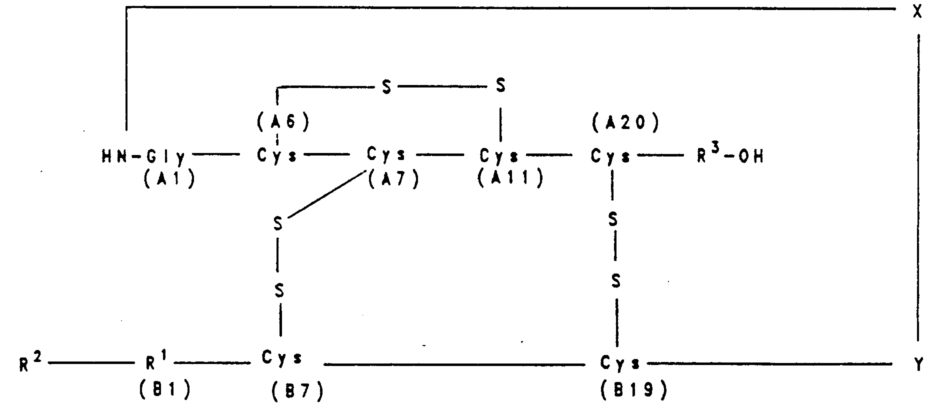 gefunden,
das dadurch gekennzeichnet ist, daß man
A) ein Protein der Formel II,

   R²-R¹-B2-B29-Y-X-Gly-A2-A20-R³ (II)

   mit einer Menge eines Mercaptans, die 2 bis 10 Reste -SH des Mercaptans pro Cysteinrest des Proteins der Formel II ergibt, in Gegenwart von einem chaotropen Hilfsstoff in einem wäßrigen Medium bei einem pH-Wert von 10 bis 11 und einer Konzentration des Proteins der Formel II von 0,05 bis 0,3 g pro Liter wäßrigen Mediums umsetzt und das erhaltene Proinsulin der Formel I
B) mit 3 bis 50 g eines hydrophoben Adsorberharzes pro Liter wäßrigen Mediums bei einem pH-Wert von 4 bis 7 versetzt,
C) das Adsorberharz mit adsorbiertem Proinsulin der Formel I isoliert und
D) das Proinsulin der Formel I vom Adsorberharz desorbiert;
dabei ist in Formel I und II
- X: a) ein genetisch kodierbarer Aminosäurerest oder
b) ein Peptid mit 2 bis 35 Aminosäureresten,
- Y: ein genetisch kodierbarer Aminosäurerest,
- R¹: ein Phenylalaninrest oder eine kovalente Bindung,
- R²: a) ein Wasserstoffatom,
b) ein genetisch kodierbarer Aminosäurerest oder
c) ein Peptid mit 2 bis 45 Aminosäureresten,
- R³: ein genetisch kodierbarer Aminosäurerest und
die Reste A2 - A20 entsprechen der Aminosäuresequenz der A-Kette von Humaninsulin, tierischem Insulin oder einem Insulinderivat und die Reste B2 - B 29 entsprechen der Aminosäuresequenz der B-Kette von Humaninsulin, tierischem Insulin oder einem Insulinderivat.

Bevorzugt sind Proteine der Formel II; dabei ist
- X: ein Peptid mit der Aminosäuresequenz der C-Kette von Humaninsulin,
- Y: ein Aminosäurerest aus der Gruppe Ala, Thr oder Ser,
- R¹: der Aminosäurerest Phe,
- R²: a) Wasserstoffatom oder
b) ein Peptid mit 2 bis 25 Aminosäureresten,
- R³: ein Aminosäurerest aus der Gruppe Asn, Ser oder Gly,
und die Reste A2 - A20 und B2 - B29 entsprechen der Aminosäuresequenz der A- und B-Ketten von Humaninsulin.

Besonders bevorzugt sind Proteine der Formel II; dabei ist
- X: ein Peptid mit 2 bis 35 Aminosäurereste, wobei am Anfang und Ende des Peptids zwei basische Aminosäurereste, insbesondere Arg und/oder Lys stehen,
- Y: der Aminosäurerest Thr,
- R¹: der Aminosäurerest Phe,
- R²: a) Wasserstoffatom oder
b) ein Peptid mit 2 bis 15 Aminosäureresten, an dessen Carboxylende der Aminosäurerest Met oder Arg steht,
- R³: der Aminosäurerest Asn,
und die Reste A2 - A20 und B2 - B29 entsprechen der Aminosäuresequenz der A- und B-Ketten von Humaninsulin.

Überraschenderweise wurde gefunden, daß das Protein der Formel II nicht in einem üblicherweise dem "Refolding" vorausgehenden Reaktionsschritt vollständig reduziert werden muß (R. Jaenicke, R. Rudolph, 1989, Folding Proteins, in Protein Structure a practical Approach; ed. Creighton, T.E. S 191-224, JRL Press Oxford; EP 0 219 874) und daß bei der beschriebenen Verfahrensweise trotz eines hohen Anteils von Fremdprotein (70 - 90 %) Faltungsausbeuten erzielt werden, die in vergleichbarer Höhe liegen wie bei der Faltung von entsprechend gereinigtem Proinsulin mit -SH-Schutzgruppen.

Das erfindungsgemäße Verfahren erlaubt, verglichen mit den bisher bekannten Methoden, eine wesentlich schnellere Herstellung mit höheren Ausbeuten. Ursache dafür ist die Vermeidung der Verfahrensschritte Sulfitolyse und gegebenenfalls Bromcyanspaltung sowie die bisher unbekannte Möglichkeit, Proinsulin entsprechend der Formel (I) direkt aus ihrem denaturierten Zustand in Gegenwart begrenzter Mengen von chaotropen Salzen in hohen Ausbeuten in die biologisch aktive Struktur zu falten. Dadurch wird es möglich, das entstandene Proinsulin durch Adsorption aus der Faltungslösung abzutrennen. So können die Proinsuline ohne Zwischenisolierung oder Reinigung nach Desorption vom Adsorptionsmittel der enzymatischen Konversion zu Insulin zugeführt werden. Das erfindungsgemäße Verfahren führt zu einem erheblich verkürzten Herstellungsverfahren, das wegen kürzerer Standzeiten und Vermeidung von Verlusten Ausbeutesteigerungen in Höhe von 25-100 % gegenüber den bekannten Verfahren ermöglicht.

Die Aminosäuresequenz von Peptiden und Proteinen wird vom N-terminalen Ende der Aminosäurekette an bezeichnet. Die in Formel I in Klammern gemachten Angaben, z.B. A6, A20, B1, B7 oder B19, entsprechen der Position von Aminosäureresten in den A- oder B-Ketten des Insulins.

Für den Begriff "genetisch kodierbarer Aminosäurerest" stehen die Aminosäuren Gly, Ala, Ser, Thr, Val, Leu, Ile, Asp, Asn, Glu, Gln, Cys, Met, Arg, Lys, His, Tyr, Phe, Trp, Pro und Selenocystein.

Für die Begriffe "Reste A2 - A20" und "Reste B2 - B 29" von tierischen Insulin werden beispielsweise die Aminosäuresequenzen von Insulin aus Rindern, Schweinen oder Hühnern verstanden.
Der Begriff Reste A2 - A20 und B2 - B29 von Insulinderivaten steht für die entsprechenden Aminosäuresequenzen von Humaninsulin, die durch den Austausch von Aminosäuren durch andere genetisch kodierbare Aminosäuren gebildet werden.

Die A-Kette von Humaninsulin hat folgende Sequenz (Seq Id No. 1):
Gly, Ile, Val, Glu, Gln, Cys, Cys, Thr, Ser, Ile, Cys, Ser, Leu, Tyr, Gln, Leu, Glu, Asn, Tyr, Cys, Asn

Die B-Kette von Humaninsulin hat folgende Sequenz (Seq Id No. 2 ):
Phe, Val, Asn, Gln, His, Leu, Cys, Gly, Ser, His, Leu, Val, Glu, Ala, Leu, Tyr, Leu, Val, Cys, Gly, Glu, Arg, Gly, Phe, Phe, Tyr, Thr, Pro, Lys, Thr

Die C-Kette von Humaninsulin hat folgende Sequenz (Seq Id No. 3).
Arg, Arg, Glu, Ala, Glu, Asp, Leu, Gln, Val, Gly, Gln, Val, Glu, Leu, Gly, Gly, Gly, Pro, Gly, Ala, Gly, Ser, Leu, Gln, Pro, Leu , Ala, Leu, Glu, Gly, Ser, Leu, Gln, Lys, Arg.

Chaotrope Hilfsmittel sind Verbindungen, die in wäßriger Lösung Wasserstoffbrückenbindungen brechen, beispielsweise Ammoniumsulfat, Guanidinhydrochlorid, Ethylencarbonat, Thiocyanat, Dimethylsulfoxid und Harnstoff.
Der Begriff hydrophobes Adsorberharz steht für nicht ionische, hydrophobe, vernetzte Polymere und/oder Copolymere, beispielsweise Polystyrol oder Copolymere aus Styrol und Divinylbenzol, insbesondere polymere Adsorberharze mit großer Oberfläche und vielen großen Poren, z.B. Handelspräparate der Firmen Rohm and Haas oder Mitsubishi Chemical Industries Ltd. wie XAD16, XAD1600 oder HP20.
Unter dem Begriff Mercaptan werden Verbindungen verstanden, die wasserlöslich sind und wenigstens eine -SH-Gruppe enthalten. Beispiele für diese Verbindungen sind Dithiothreitol, Dithioerythrol, 2-Mercaptoethanol, Cystein, Methylthioglykolat, 3-Mercapto-1,2-propandiol und 3-Mercaptopropionsäure.

Das Protein der Formel II kann mit Hilfe einer Vielzahl gentechnischer Konstrukte in Mikroorganismen gebildet werden (EP 0 489 780, EP 0 347 781, EP 0 453 969). Die gentechnischen Konstrukte werden in Mikroorganismen wie Escherichia coli oder Streptomyceten während der Fermentation exprimiert. Die gebildeten Proteine werden im Inneren der Mikroorganismen abgelagert (EP 0 489 780) oder in die Fermentationslösung ausgeschieden.

Für das erfindungsgemäße Verfahren können Proteine der Formel II eingesetzt werden, die direkt nach dem Zellaufschluß noch mit einer Vielzahl von Proteinen, die aus der Fermentationslösung und aus den Mikroorganismen stammen, verunreinigt sind. Die Proteine der Formel II können aber auch in vorgereinigter Form beispielsweise nach einer Fällung oder einer chromatographischen Reinigung eingesetzt werden.

Beim Verfahrensschritt A) geht man wie folgt vor:
Die Proteine werden in einem chaotropen Hilfsmittel oder in Mischungen von verschiedenen chaotropen Hilfsmitteln gelöst. Bevorzugt wird Guanidinhydrochlorid in einer Konzentration von 6 bis 10 M, bevorzugt 8 M, bezogen auf Wasser als Lösungsmittel, verwendet. Der pH-Wert des Reaktionsgemischs beträgt 8,5 bis 10,8. Beispiele für brauchbare Puffer sind Phosphat-, Tri(hydroxymethyl)aminomethan(Tris)-, Borat- oder Glycinpuffer. Die Konzentration der Puffersubstanzen im wäßrigem Medium beträgt bis zu 0,5 M, bevorzugt von 0,005 M bis 0,5 M, besonders bevorzugt von 0,05 bis 0,1 M. Anschließend wird die Proteinmischung mit einer wäßrigen Mercaptanlösung gemischt. Dadurch werden folgende Konzentrationen in der Reaktionslösung (bezogen auf Wasser) eingestellt:

Die Konzentration des Proteins der Formel II beträgt 0,05 bis 0,3 g/l, bevorzugt 0,1 bis 0,2 g/l.

Die Menge des Mercaptans beträgt 2 bis 10 Reste -SH des Mercaptans pro Cysteinrest des Proteins der Formel II, bevorzugt 3 bis 6.
Der pH-Wert der Lösung beträgt 10 bis 11, bevorzugt 10,8. Es werden die obengenannten Pufferkomponenten verwendet. Der genaue pH-Wert wird durch Zugabe von Natronlauge eingestellt. Die Konzentration der Pufferkomponenten beträgt 0,005 bis 0,5 M, bevorzugt 0,05 bis 0,1 M.

Die Konzentration des chaotropen Hilfsmittels im Reaktionsansatz mit dem Mercaptan beträgt weniger als 1 M, bevorzugt 0,1 bis 0,8 M, insbesondere 0,3 bis 0,6 M. Als Mercaptan wird bevorzugt Cystein oder 2-Mercaptoethanol allein oder als Mischung eingesetzt.

Die Temperatur während der Faltung im Verfahrensschritt A) beträgt 0° C bis 50 °C, vorzugsweise 2° C bis 30 °C, insbesondere 4 °C bis 10 °C. Die Reaktionszeit beträgt 3 bis 12 Stunden, bevorzugt 4 bis 8 Stunden, insbesondere 5 Stunden.

Das Ergebnis von Verfahrensschritt A) ist ein Proinsulin der Formel I, welches korrekt verbundene Cystinbrücken enthält.

Bei Verfahrensschritt B) wird die Reaktionslösung aus Verfahrensschritt A) mit einer Säure wie Salzsäure oder Schwefelsäure auf einen pH-Wert von 4 bis 7 eingestellt. Wenn es zu Trübungen der Lösung kommen sollte, werden diese durch Filtration oder Zentrifugation entfernt. Die verbliebene Lösung wird mit einem hydrophoben Adsorberharz versetzt. Als günstig haben sich Harze vom Typ des XAD oder HP20 bewährt. Die Menge des Harzes beträgt 3 bis 50 g pro Liter Reaktionslösung, bevorzugt 10 bis 25 g/l.

Die erhaltene Suspension wird 3 bis 4 Stunden gerührt. Die Adsorption des Proinsulins der Formel I an das Harz wird durch Probennahme und Hochdruckflüssigchromatographie-Analyse (HPLC-Analyse) kontrolliert. Sobald kein Proinsulin mehr in der Lösung nachweisbar ist, wird das Adsorberharz von der wäßrigen Reaktionslösung abgetrennt (Verfahrensschritt C). Dies erfolgt beispielsweise durch Filtration oder Zentrifugation nach bekannten Methoden. Das Harz mit adsorbiertem Proinsulin wird mit einer rein wäßrigen oder pufferhaltigen wäßrigen Lösung gewaschen.

Die Desorption (Verfahrensschritt D) des Proinsulins der Formel I erfolgt nach bekannten Methoden, die vom verwendeten hydrophoben Adsorberharz abhängen. Die Desorption erfolgt bei XAD- oder HP20-Adsorberharzen beispielsweise durch eine wäßrige Lösung, die 10 bis 50 % eines mit Wasser mischbaren, nichtionischen organischen Lösungsmittels, z.B. ein (C₁-C₆)-Alkanol, enthält. Bevorzugt wird 50 % Isopropanol im Lösungsmittel Wasser verwendet. Die Desorption des Proinsulins der Formel I erfolgt beispielsweise durch Waschen mit der Isopropanollösung. Das Waschen kann in einem Rührkessel durch Mischen mit der Isopropanollösung erfolgen oder durch Chromatographie in einer Säule. Das Volumenverhältnis Harz zu Isopropanollösung beträgt 1:1 bis 1:10 bevorzugt 1:1,1 bis 1:2. Die Waschschritte können ein- bis fünfmal wiederholt werden, bevorzugt zweimal. Die vereinigten Isopropanolfraktionen können direkt oder nach Verdünnung mit Wasser für weitere Chromatographiereinigungen oder für die enzymatische Spaltung des Proinsulins zu Insulin verwendet werden (Kemmler et al., J. Biol. Chem. 246 (1971) Seite 6786; EP 0 305 760).

In den folgenden Beispielen wird das erfindungsgemäße Verfahren im einzelnen beschrieben. Prozentangaben beziehen sich auf das Gewicht, sofern nicht anders angegeben.

### Beispiel 1

Durch Fermentation von genetisch modifizierten Escherichia-coli-Zellen (EP 0 489 780) wird Proinsulin 1 mit folgender Aminosäuresequenz hergestellt. Proinsulin 1 (Seq Id No. 4):
Met Ala Thr Thr Ser Thr Gly Asn Ser Ala Arg Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn

Proinsulin 1 entspricht der Formel II, dabei ist
- X: C-Peptid von Humaninsulin,
- Y: Thr (B30),
- R¹: Phe (B1),
- R²: ein Peptid mit 11 Aminosäureresten,
- R³: Asn (A21) und

A2 - A20 die Aminosäuresequenz der A-Kette von Humaninsulin (Aminosäurereste 2 bis 20) und B2 - B29 die Aminosäuresequenz der B-Kette von Humaninsulin (Aminosäurereste 2 bis 29).

In den E.-coli-Zellen sammelt sich das exprimierte Proinsulin 1 und bildet Einschlußkörper. Nach Beendigung der Fermentation werden die Zellen durch Zentrifugation abgetrennt und durch übliche Hochdruckhomogenisation aufgeschlossen. Die freigesetzten Fusionsproteineinschlußkörperchen werden durch Zentrifugation isoliert.

1000 g der isolierten Fusionsproteineinschlußkörper (bezogen auf Trockenmasse nach Gefriertrocknung) des Proinsulins werden in 100 l einer 8 M Guanidinhydrochlorid-Lösung bei pH 10,8 gelöst. Nach Zentrifugation geringer Mengen Trübstoffe wird die klare Lösung in 900 l einer wäßrigen Cysteinlösung (125 g Cysteinhydrochloridhydrat) bei einem pH-Wert von 10,8 und einer Temperatur von 4 °C eingerührt. Der Anteil des insulinhaltigen Fusionproteins wird mit Hilfe von SDS-Elektrophorese-Scan (U.K. Lämli, Nature, Band 227, Seiten 680-685, 1970) bestimmt. Er beträgt 15 %.
Nach Abschluß der Faltungsreaktion wird mit Hilfe der analytischen HPLC ein Proinsulin-Gehalt im Reaktionsansatz von 105 g gemessen.
Die Lösung wird mit 6 N HCl auf einen pH von 4,5 eingestellt und eine leichte Trübung durch Zentrifugation entfernt. Zur klaren Lösung werden 30 kg von HP20 (Mitsubishi Chemical Industries Ltd., Düsseldorf, Deutschland) gegeben. Die Suspension wird etwa 4 Stunden langsam gerührt, bis kein Proinsulin 1 mehr im Überstand nachzuweisen ist. Durch Filtration über eine Nutsche wird das Harz abgetrennt und mit Wasser gewaschen. Die Desorption des Produktes erfolgt durch Aufschlämmung des Harzes in 50 l 50%iger, wäßriger Isopropanollösung. Filtration und Inkubation mit der Isopropanollösung wird zweimal wiederholt. Die Ausbeute an Proinsulin der Formel I beträgt 103 g.

### HPLC-Analyse

0,5 g Protein werden in 40 ml einer Lösung aus 6 M Guanidinhydrochlorid, 50 mM Tris, pH 8,5, 5 mM Ethylendiamintetraacetat (EDTA), 1 % 2-Mercaptoethanol, 10 mM Dithiothreitol bei 95 °C für 2 min gelöst und dann bei 14000 g für 20 min zentrifugiert. Von dem klaren Überstand werden 0,02 ml auf eine Hochdruckflüssigchromatographiesäule aufgetragen.
- Säule:: ®Nucleogel RP 300-5/46 (Macherey & Nagel, Aachen, Deutschland)
- Gradient:: Puffer A: 0,1 % Trifluoressigsäure (TFA) Puffer B: 0,09 % TFA in Acetonitril
- Temperatur:: 55 °C
- Gesamtlaufzeit:: 15 min,

Der Gradient ist gekennzeichnet durch die folgende Mengen von Puffer B nach den entsprechenden Laufzeiten :
10 min 25%, 12 min 60 %, 13 min 90 %, 15 min 100 %.
- Fluß:: 1 ml/min
- Detektion:: 215 nm

Retentionszeit von
- Proinsulin:: 9 min.

### Beispiel 2

Durch Fermentation von einem genetisch modifizierten E. coli (EP 0 347 781) wird ein Proinsulin 2 mit folgender Aminosäuresequenz hergestellt:
Proinsulin 2 (Seq Id No:. 5)
Met Ala Pro Thr Ser Ser Ser Thr Lys Lys Thr Gln Leu Gln Leu Glu His Leu Leu Leu Asp Leu Gln Met Ile Leu Asn Gly Ile Asn Asn Tyr Lys Asn Pro Lys Leu Thr Arg Met Ile Glu Gly Arg Phe Val Asn Gln His Leu Cys Gly Ser His Leu Val Glu Ala Leu Tyr Leu Val Cys Gly Glu Arg Gly Phe Phe Tyr Thr Pro Lys Thr Arg Gly Ile Val Glu Gln Cys Cys Thr Ser Ile Cys Ser Leu Tyr Gln Leu Glu Asn Tyr Cys Asn

In den E.-coli-Zellen sammelt sich das exprimierte Proinsulin 2 und bildet häufig Einschlußkörper. Der Aufschluß der Zellen erfolgt wie in Beispiel 1. Die so erhaltenen Proinsuline werden einer Bromcyanspaltung unterworfen und es entsteht Proinsulin 3 (Seq Id No. 7).
Proinsulin 3 entspricht der Formel II, dabei ist
- X: Arg,
- Y: Thr (B30),
- R¹: Phe (B1),
- R²: ein Peptid mit 4 Aminosäureresten,
- R³: Asn (A21) und

A2 - A20 und B2 - B29 entsprechen der Aminosäuresequenz der A- und B-Ketten von Humaninsulin.

Nach der Bromcyanspaltung wird der Gehalt an Proinsulin 3 mit Hilfe der SDS-Elektrophorese in einer gefriergetrockneten Probe mit 9,2 % insulinhaltiges Protein quantitativ bestimmt.
1000 g des Proinsulin 3 werden wie im Beispiel 1 mit Cysteinhydrochloridhydrat inkubiert. Danach wird mittels analytische HPLC ein Gehalt von 63 g an Proinsulin der Formel I in dem gesamten Reaktionsansatz bestimmt. Wie in Beispiel 1 wird das Produkt mit 35 kg XAD-1600 Harz (Rohm and Haas Company, Philadelphia, USA) adsorbiert und aus der Reaktionslösung entfernt. Nach Waschen des Harzes und Desorption des Produktes wird der Gehalt an Proinsulin der Formel I mit 56 g bestimmt.

### Beispiel 3

Durch Fermentation von einem gentechnisch modifizierten E. coli (EP 0 489 780) wird Proinsulin 4 mit folgender Aminosäuresequenz hergestellt:
Proinsulin 4 (Seq Id No: 6)
Met Ala Thr Thr Ser Thr Gly Asn Ser Ala Arg Phe Val Asn Gln His Leu Cys Gly Ser His Leu Val Glu Ala Leu Tyr Leu Val Cys Gly Glu Arg Gly Phe Phe Tyr Thr Pro Lys Thr Arg Gly Ile Val Glu Gln Cys Cys Thr Ser Ile Cys Ser Leu Tyr Gln Leu Glu Asn Tyr Cys Asn

Proinsulin 4 entspricht der Formel II, dabei ist
- X: Arg,
- Y: Thr, (B30),
- R¹: Phe (B1),
- R²: ein Peptid mit 11 Aminosäuren,
- R³: Asn (A21) und

A2 - A20 und B2 - B29 entsprechen der Aminosäuresequenz der A- und B-Ketten von Humaninsulin.

Nach Aufschluß der Zellen wird aus dem Homogenisat das Fusionsprotein durch Zentrifugation gewonnen. Davon werden nach Bestimmung des Produktgehaltes (15 %) wie in Beispiel 1 1000 g zur Faltungsreaktion eingesetzt. Die Faltungsausbeute am Ende der Reaktion wird wie in Beispiel 1 mit 60 % bestimmt. Die Ausbeute beträgt 108 g Proinsulin.

Sequenzprotokoll

SEQ ID NO:1
Art der Sequenz: Aminosäure (AS)
Sequenzlänge: 21 AS

Gly Ile Val Glu Gln Cys Cys Thr Ser Ile Cys Ser Leu Tyr Gln Leu
1               5                   10                  15
Glu Asn Tyr Cys Asn
            20

SEQ ID NO:2
Art der Sequenz: AS
Sequenzlänge: 30 AS

Phe Val Asn Gln His Leu Cys Gly Ser His Leu Val Glu Ala Leu Tyr
1               5                   10                  15
Leu Val Cys Gly Glu Arg Gly Phe Phe Tyr Thr Pro Lys Thr
            20                  25                  30

SEQ ID NO:3
Art der Sequenz: Aminosäure (AS)
Sequenzlänge: 35 AS

Arg Arg Glu Ala Glu Asp Leu Gln Val Gly Gln Val Glu Leu Gly Gly
1               5                   10                  15
Gly Pro Gly Ala Gly Ser Leu Gln Pro Leu Ala Leu Glu Gly Ser Leu
            20                  25                  30
Gln Lys Arg
        35

SEQ ID NO:4
Art der Sequenz: Aminosäure (AS)
Sequenzlänge: 97 AS

Met Ala Thr Thr Ser Thr Gly Asn Ser Ala Arg Phe Val Asn Gln His
1               5                   10                  15
Leu Cys Gly Ser His Leu Val Glu Ala Leu Tyr Leu Val Cys Gly Glu
            20                  25                  30
Arg Gly Phe Phe Tyr Thr Pro Lys Thr Arg Arg Glu Ala Glu Asp Leu
        35                  40                  45
Gln Val Gly Gln Val Glu Leu Gly Gly Gly Pro Gly Ala Gly Ser Leu
    50                  55                  60
Gln Pro Leu Ala Leu Glu Gly Ser Leu Gln Lys Arg Gly Ile Val Glu
65                  70                  75                  80
Gln Cys Cys Thr Ser Ile Cys Ser Leu Tyr Gln Leu Glu Asn Tyr Cys
                85                  90                  95
Asn

SEQ ID NO:5
Art der Sequenz: AS
Sequenzlänge: 96 AS

Met Ala Pro Thr Ser Ser Ser Thr Lys Lys Thr Gln Leu Gln Leu Glu
1               5                   10                  15
His Leu Leu Leu Asp Leu Gln Met Ile Leu Asn Gly Ile Asn Asn Tyr
            20                  25                  30
Lys Asn Pro Lys Leu Thr Arg Met Ile Glu Gly Arg Phe Val Asn Gln
        35                  40                  45
His Leu Cys Gly Ser His Leu Val Glu Ala Leu Tyr Leu Val Cys Gly
    50                  55                  60
Glu Arg Gly Phe Phe Tyr Thr Pro Lys Thr Arg Gly Ile Val Glu Gln
65                  70                  75                  80
Cys Cys Thr Ser Ile Cys Ser Leu Tyr Gln Leu Glu Asn Tyr Cys Asn
                85                  90                  95

SEQ ID NO:6
Art der Sequenz: Aminosäure (AS)
Sequenzlänge: 63 AS

Met Ala Thr Thr Ser Thr Gly Asn Ser Ala Arg Phe Val Asn Gln His
1               5                   10                  15
Leu Cys Gly Ser His Leu Val Glu Ala Leu Tyr Leu Val Cys Gly Glu
            20                  25                  30
Arg Gly Phe Phe Tyr Thr Pro Lys Thr Arg Gly Ile Val Glu Gln Cys
        35                  40                  45
Cys Thr Ser Ile Cys Ser Leu Tyr Gln Leu Glu Asn Tyr Cys Asn
    50                  55                  60

SEQ ID NO: 7
Art der Sequenz: AS
Sequenzlänge: 56 AS

Ile Glu Gly Arg Phe Val Asn Gln His Leu Cys Gly Ser His Leu Val
1               5                   10                  15
Glu Ala Leu Tyr Leu Val Cys Gly Glu Arg Gly Phe Phe Tyr Thr Pro
            20                  25                  30
Lys Thr Arg Gly Ile Val Glu Gln Cys Cys Thr Ser Ile Cys Ser Leu
        35                  40                  45
Tyr Gln Leu Glu Asn Tyr Cys Asn
    50                  55

## Patentansprüche

1. Verfahren zur Gewinnung von Proinsulin der Formel I 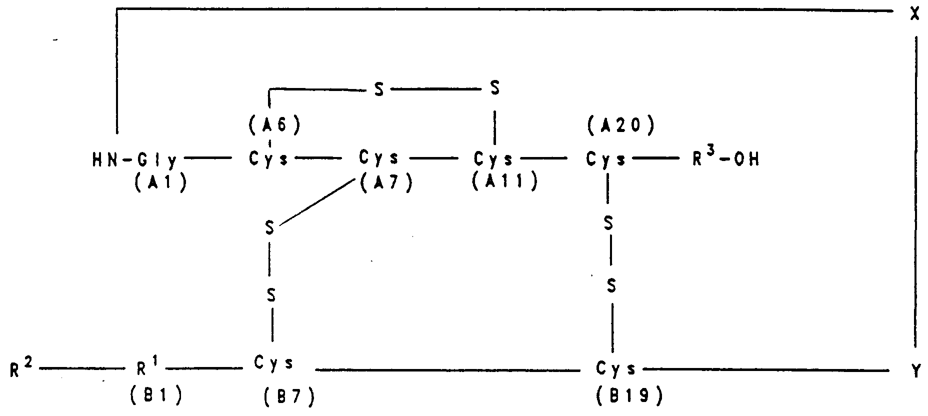 dadurch gekennzeichnet, daß man
A) ein Protein der Formel II,
R²-R¹-B2-B29-Y-X-Gly-A2-A20-R³ (II)
mit einer Menge eines Mercaptans, die 2 bis 10 Reste -SH des Mercaptans pro Cysteinrest des Proteins der Formel II ergibt, in Gegenwart von mindestens einem chaotropen Hilfsstoff in einem wäßrigen Medium bei einem pH-Wert von 10 bis 11 und einer Konzentration des Proteins der Formel II von 0,05 bis 0,3 g pro Liter wäßrigen Mediums umsetzt und das erhaltene Proinsulin der Formel I
B) mit 3 bis 50 g eines hydrophoben Adsorberharzes pro Liter wäßrigen Mediums bei einem pH-Wert von 4 bis 7 versetzt,
C) das Adsorberharz mit adsorbiertem Proinsulin der Formel I isoliert und
D) das Proinsulin der Formel I von Adsorberharz desorbiert;
dabei ist in Formel I und II
X
a) ein genetisch kodierbarer Aminosäurerest oder
b) ein Peptid mit 2 bis 35 Aminosäureresten,
Y ein genetisch kodierbarer Aminosäurerest,
R¹ ein Phenylalaninrest oder eine kovalente Bindung,
R²
a) ein Wasserstoffatom,
b) ein genetisch kodierbarer Aminosäurerest oder
c) ein Peptid mit 2 bis 45 Aminosäureresten,
R³ ein genetisch kodierbarer Aminosäurerest und
die Reste A2 - A20 entsprechen der Aminosäuresequenz der A-Kette von Humaninsulin, tierischem Insulin oder einem Insulinderivat und die Reste B2 - B 29 entsprechen der Aminosäuresequenz der B-Kette von Humaninsulin, tierischem Insulin oder einem Insulinderivat.

2. Verfahren nach Anspruch 1, dadurch gekennzeichnet, daß ein Protein der Formel II eingesetzt wird, dabei ist
X ein Peptid mit der Aminosäuresequenz der C-Kette von Humaninsulin,
Y ein Aminosäurerest aus der Gruppe Ala, Thr oder Ser,
R¹ der Aminosäurerest Phe,
R²
a) Wasserstoffatom oder
b) ein Peptid mit 2 bis 25 Aminosäureresten,
R³ ein Aminosäurerest aus der Gruppe Asn, Ser oder Gly,
und die Reste A2 - A20 und B2 - B29 entsprechen der Aminosäuresequenz der A- und B-Ketten von Humaninsulin.

3. Verfahren nach Anspruch 1 oder 2, dadurch gekennzeichnet, daß ein Protein der Formel II eingesetzt wird, dabei ist
X ein Peptid mit 2 bis 35 Aminosäurereste, wobei am Anfang und Ende des Peptids zwei basische Aminosäurereste, insbesondere Arg und/oder Lys stehen,
Y der Aminosäurerest Thr,
R¹ der Aminosäurerest Phe,
R²
a) Wasserstoffatom oder
b) ein Peptid mit 2 bis 15 Aminosäureresten, an dessen Carboxylende der Aminosäurerest Met oder Arg steht,
R³ der Aminosäurerest Asn,
und die Rest A2 - A20 und B2 - B29 entsprechen der Aminosäuresequenz der A- und B-Ketten von Humaninsulin.

4. Verfahren nach einem oder mehreren der Ansprüche 1 bis 3, dadurch gekennzeichnet, daß als Mercaptan Cystein oder Cysteinhydrochloridhydrat eingesetzt wird.

5. Verfahren nach einem oder mehreren der Ansprüche 1 bis 4, dadurch gekennzeichnet, daß als hydrophobes Adsorberharz ein vernetztes Polystyrol oder ein Copolymer aus Polystyrol und Divinylbenzyl eingesetzt wird.

6. Verfahren nach einem oder mehreren der Ansprüche 1 bis 5, dadurch gekennzeichnet, daß das Proinsulin der Formel I vom Adsorberharz mit Isopropanol desorbiert wird.

## Claims

1. A process for obtaining proinsulin of the formula I 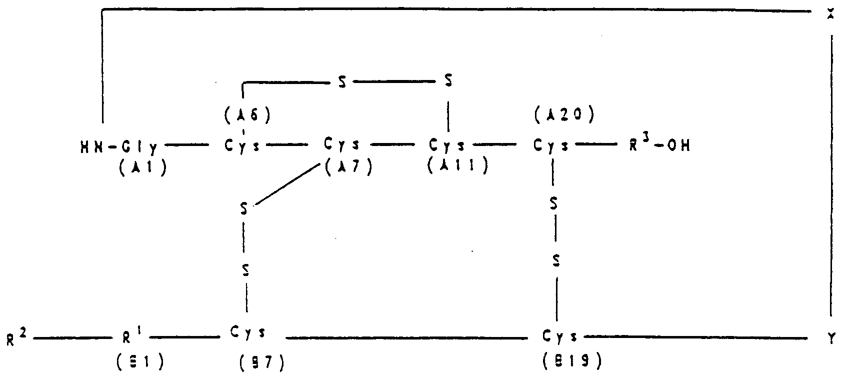 which comprises
A) reacting a protein of the formula II,
R²-R¹-B2-B29-Y-X-Gly-A2-A20-R³ (II)
with a quantity of a mercaptan, which quantity yields 2 to 10 -SH radicals of the mercaptan per cysteine residue of the protein of the formula II, in the presence of at least one chaotropic auxiliary agent in an aqueous medium at a pH of 10 to 11 and at a concentration of the protein of the formula II of 0.05 to 0.3 g per liter of aqueous medium, and the proinsulin of the formula I which is obtained
B) being mixed with 3 to 50 g of a hydrophobic adsorber resin per liter of aqueous medium at a pH of 4 to 7,
C) the adsorber resin; which has adsorbed proinsulin of the formula I, being isolated, and
D) the proinsulin of the formula I being desorbed from the adsorber resin;
in formula I and II
X is
a) a genetically encodable amino acid residue or
b) a peptide possessing 2 to 35 amino acid residues,
Y is a genetically encodable amino acid residue,
R¹ is a phenylalanine residue or a covalent bond,
R² is
a) a hydrogen atom,
b) a genetically encodable amino acid residue or
c) a peptide possessing 2 to 45 amino acid residues,
R³ is a genetically encodable amino acid residue, and
the residues A2 - A20 correspond to the amino acid sequence of the A chain of human insulin, animal insulin, or an insulin derivative, and the residues B2 - B29 correspond to the amino acid sequence of the B chain of human insulin, animal insulin, or an insulin derivative.

2. The process as claimed in claim 1, wherein a protein of the formula II is employed in which
X is a peptide possessing the amino acid sequence of the C chain of human insulin,
Y is an amino acid residue selected from the group comprising Ala, Thr or Ser,
R¹ is the amino acid residue Phe,
R² is
a) a hydrogen atom, or
b) a peptide possessing 2 to 25 amino acid residues,
R³ is an amino acid residue selected from the group comprising Asn, Ser or Gly,
and the residues A2 - A20 and B2 - B29 correspond to the amino acid sequence of the A and B chains of human insulin.

3. The process as claimed in claim 1 or 2, wherein a protein of the formula II is employed in which
X is a peptide possessing 2 to 35 amino acid residues, where two basic amino acid residues, in particular Arg and/or Lys, are located at the beginning and at the end of the peptide,
Y is the amino acid residue Thr,
R¹ is the amino acid residue Phe,
R² is
a) a hydrogen atom, or
b) a peptide possessing 2 to 15 amino acid residues, at whose carboxyl end the amino acid residue Met or Arg is located,
R³ is the amino acid residue Asn,
and the residues A2 - A20 and B2 - B29 correspond to the amino acid sequence of the A and B chains of human insulin.

4. The process as claimed in one or more of claims 1 to 3, wherein cysteine or cysteine hydrochloride hydrate is employed as the mercaptan.

5. The process as claimed in one or more of claims 1 to 4, wherein a crosslinked polystyrene or a copolymer composed of polystyrene and divinylbenzyl is employed as the hydrophobic adsorber resin.

6. The process as claimed in one or more of claims 1 to 5, wherein the proinsulin of the formula I is desorbed from the adsorber resin using isopropanol.

## Revendications

1. Procédé de préparation de proinsuline de formule I 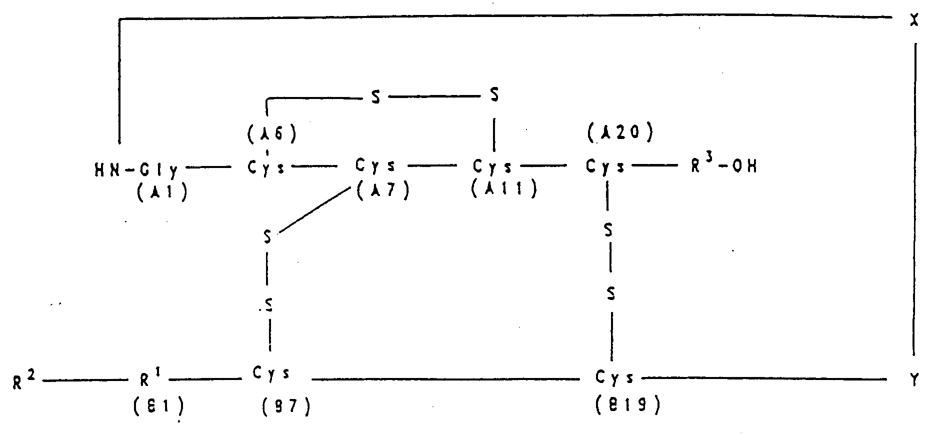 caractérisé en ce que
A) on fait réagir une protéine de formule II
R²-R¹-B2-B29-Y-X-Gly-A2-A20-R³ (II)
avec une quantité d'un mercaptan qui correspond à 2 à 10 groupes -SH du mercaptan par groupe cystéine de la protéine de formule II, en présence d'au moins un additif chaotrope, dans un milieu aqueux à un pH de 10 à 11 et à une concentration de protéine de formule II de 0,05 à 0,3 g par litre de milieu aqueux et que
B) on met en présence la proinsuline de formule I obtenue avec 3 à 50 g d'une résine d'adsorption hydrophobe par litre de milieu aqueux à un pH de 4 à 7,
C) on isole la résine d'adsoption avec la proinsuline de formule I adsorbée et
D) on désorbe la proinsuline de formule I de la résine d'adsorption;
avec les signifcations suivantes pour les formules I et II
X
a) un reste d'un aminoacide génétiquement codable ou
b) un peptide formé de 2 à 35 restes d'aminoacides,
Y un reste d'un aminoacide génétiquement codable,
R¹ un reste de phénylalanine ou une liaison covalente,
R²
a) un atome d'hydrogène,
b) un reste d'un aminoacide génétiquement codable ou
c) un peptide formé de 2 à 45 restes d'aminoacides,
R³ un reste d'aminoacide génétiquement codable et
les groupes A2 - A20 correspondent à la séquence d'aminoacides de la chaîne A d'insuline humaine, d'insuline animale ou d'un dérivé d'insuline et les groupes B2 - B29 correspondent à la séquence d'aminoacides de la chaîne B d'insuline humaine, d'insuline animale ou d'un dérivé d'insuline.

2. Procédé selon la revendication 1, caractérisé en ce que l'on utilise une protéine de formule II, dans laquelle
X est un peptide dont la séquence d'aminoacides correspond à la chaîne C
de l'insuline humaine,
Y un reste d'un aminoacide du groupe Ala, Thr ou Ser,
R¹ le reste de l'aminoacide Phe,
R²
a) un atome d'hydrogène ou
b) un peptide formé de 2 à 25 restes d'aminoacides,
R³ un reste d'un aminoacide du groupe Asn, Ser ou Gly,
et les groupes A2 - A20 et B2 - B29 correspondent aux séquences d'aminoacides des chaînes A et B de l'insuline humaine.

3. Procédé selon la revendication 1 ou 2, caractérisé en ce que l'on utilise une protéine de formule II, dans laquelle
X est un peptide formé de 2 à 35 restes d'aminoacides, la chaîne peptidique
portant au début et à la fin deux restes d'aminoacides basiques, en particulier Arg et/ou Lys,
Y le reste de l'aminoacide Thr,
R¹ le reste de l'aminoacide Phe,
R²
a) un atome d'hydrogène ou
b) un peptide formé de 2 à 15 restes aminoacides dont l'extrémité carboxylique porte le reste de l'aminoacide Met ou Arg,
R³ le reste de l'aminoacide Asn,
et les groupes A2 - A20 et B2 - B29 correspondent aux séquences d'aminoacides des chaînes A et B de l'insuline humaine.

4. Procédé selon une ou plusieurs des revendications 1 à 3, caractérisé en ce que l'on utilise la cystéine ou le chlorhydrate hydraté de cystéine comme mercaptan.

5. Procédé selon une ou plusieurs des revendications 1 à 4, caractérisé en ce que l'on utilise un polystyrène réticulé ou un copolymère de polystyrène et de divinylbenzène comme résine d'adsoption hydrophobe.

6. Procédé selon une ou plusieurs des revendications 1 à 5, caractérisé en ce que la proinsuline de formule I est désorbée de la résine d'adsorption par l'isopropanol.
